# EUROPEAN PATENT SPECIFICATION

(11) **EP 2 948 133 B1**
(45) Date of publication and mention of the grant of the patent: **07.09.2016**
(21) Application number: 14704090.1
(22) Date of filing: 27.01.2014
(51) Int. Cl.: A61K 9/24, A61K 9/20, A61K 31/19, A61K 31/70

(54) **PHARMACEUTICAL COMBINATIONS OF FLURBIPROFEN, GLUCOSAMIN AND CAPSAICIN**
PHARMAZEUTISCHE KOMBINATIONEN AUS FLURBIPROFEN, GLUCOSAMIN UND CAPSAICIN
NOUVELLES COMPOSITIONS PHARMACEUTIQUES DE FLURBIPROFÈNE GLUCOSAMINE ET CAPSAICIN

(30) Priority: 28.01.2013 TR 201301016; 24.01.2014 TR 201400851
(43) Date of publication of application: 02.12.2015
(73) Proprietor: Sanovel Ilac Sanayi ve Ticaret A.S., 34460 Istanbul (TR)
(72) Inventor: CIFTER, Ümit, 34460 Istanbul (TR); MUTLU, Onur, 34460 Istanbul (TR); TÜRKYILMAZ, Ali, 34460 Istanbul (TR); RAMAZANOGLU, Gaye, 34460 Istanbul (TR)
(74) Representative: Sevinç, Erkan
(86) International application number: PCT/EP2014/051500
(87) International publication number: WO 2014/114786

(56) References cited:
- WO-A2-2008/008474
- US-A1- 2004 152 713
- US-A1- 2009 074 889

## Description

### Field of Invention

The present invention relates to a pharmaceutical combination of flurbiprofen or a pharmaceutically acceptable salt thereof, glucosamine or a pharmaceutically acceptable salt thereof and capsaicin or a pharmaceutically acceptable salt thereof. Particularly, the present invention relates to a pharmaceutical combination for use in the treatment of pain and inflammatory symptoms associated with joint and cartilage disorders, especially with osteoarthritis and rheumatoid arthritis.

### Background of Invention

Joint and cartilage disorders, is a painful degenerative condition that results in the deterioration of cartilage tissues that support the weight-bearing joints in the body. Once the cartilage is thinned or lost, the constant grinding of bones against each other causes pain and stiffness around the joint. Abnormal and excess bone formations called spurs grow from the damaged bones, causing further pain and stiffness. It is believed that degenerative joint disorders affect 80% of people over the age of 60. Degenerative joint disorders include, for example, osteoarthritis, rheumatoid arthritis, other rheumatic disorders with cartilage breakdown, chondrolysis after joint trauma, for example, after meniscus or patella injuries or torn ligaments, or chondrolysis associated with prolonged immobilization of joints.

**Osteoarthritis** is the most prevalent form of arthritis which is a painful, degenerative joint disease that often involves the hips, knees, neck, lower back, or the small joints of the hands. It is characterized by pain and progressive degeneration of cartilage in synovial joints and vertebrae, leading to significant reduction of mobility and quality of life. Osteoarthritis usually develops in joints that are injured by repeated overuse in the performance of a particular job or a favorite sport or from carrying around excess body weight. Eventually this injury or repeated impact thins or wears away the cartilage that cushions the ends of the bones in the joint so that the bones rub together, causing a grating sensation. Joint flexibility is reduced, bony spurs develop, and the joint swells. Usually, the first symptom a person has with osteoarthritis is pain that worsens following exercise or immobility.

Rheumatoid arthritis is an autoimmune inflammatory disease in which the body releases enzymes that attack its own healthy tissues. In rheumatoid arthritis, these enzymes destroy the linings of joints causing pain, swelling, stiffness, deformity, and reduced movement and function. Rheumatoid arthritis also may include systemic symptoms.

Hence, pharmacological treatment of arthritis involves two therapeutic goals:
- Analgesic & anti-inflammatory treatment: Relief from pain and inflammation of the soft tissue surrounding the joint.
- Disease-modifying treatment to treat the underlying pathology.

Flurbiprofen is a well-known, propionic acid derivative, also known as NSAID (non-steroidal anti-inflammatory drug), with the analgesic and anti-inflammatory activities it possesses. It is used in muscle-skeletal and joint disorders such as ankylosing spondylitis, osteoarthritis and rheumatoid arthritis, in soft-tissue disorders such as sprains and strains and for postoperative pains and mild to moderate pain including dysmenorrhoea and migraine. Its chemical structure is illustrated with Formula I given below.

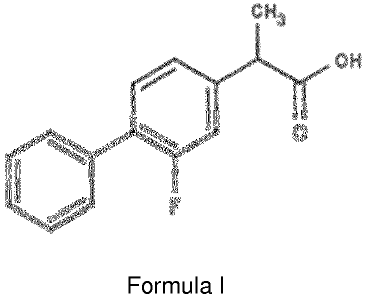

Flurbiprofen is mostly administrated orally in dosages about 150 to 200 mg, may also be increased to 300 mg daily in acute or severe conditions if necessary.

One disadvantage of the oral administration of combinations comprising flurbiprofen, is that the patient is likely to experience unpleasant side effects, including gastrointestinal (GI) adverse effects including inflammation, spontaneous gastric bleeding, ulceration and perforation of the stomach, which can be life threatening. Thus, using flurbiprofen in high dosages may increase the GI adverse effects.

There are various patent applications in prior art in relation to flurbiprofen combinations, for example, US3755427 describes the flurbiprofen molecule and the anti-inflammatory, analgesic, antipyretic and anti-toxic effects of flurbiprofen.

The document EP137668 discloses the use of flurbiprofen in the treatment of alveolar bone resorption.

Glucosamine is an amino sugar and prominent precursor in the biochemical synthesis of glycosylated proteins and lipids. Glucosamine is part of the structure of the polysaccharides chitosan and chitin and it is naturally present in the shells of shellfish, animal bones and bone marrow. It is also present in some fungi and can be also synthetically derived. Glucosamine is used for the treatment of osteoarthritis. Glucosamine may be administered in dosages about 500 to 2500 mg per day.

Capsaicin (trans-8-methyl-N-vanillyl-6-nonenamide, formula II) is the main pungent ingredient of hot red and chili peppers that belong to the plant genus Capsicum (Solanaceae). Capsaicin carries analgesic properties. It is a TRPV1 channel agonist indicated for the management of neuropathic pain associated with postherpetic neuralgia. It triggers the release of endorphins from the pituitary gland and hypothalamus which helps relieve muscle pain and joint pain associated with arthritis, simple backaches, sprains, strains, and bruises. It is marketed as a cutaneous patch under the name of Qutenza. In this present invention, capsaicin has been used in a combination with flurbiprofen and glucosamine for the oral administration. Although oral administration of capsaicin has more advantageous than the topical administration over the ease of use, it has also a drawback such as salty and bitter pepper taste. Therefore, for the convenience of patients, it is needed to overcome this effect of capsaicin on formulation taste.

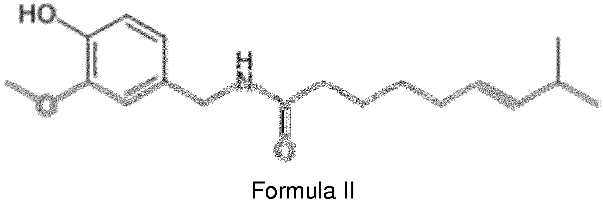

There are various patent applications in prior art in relation to glucosamine, flurbiprofen and capsaicin active agents but none of them are specifically used in combination with flurbiprofen, glucosamine and capsaicin in oral administration as tablet or capsule dosage form.

It is well known that drugs used in the same therapeutic area or even for treating the same indication cannot always be combined *a priori* with the expectation of at least additive therapeutic effects. The scientific literature is full of examples wherein compounds of different classes, which are used to treat the same indications, cannot be combined into safe and efficacious dosage forms thereby resulting in incompatible drug combinations. The reasons for this unexpected lack of compatibility are varied; however, it is often found that the incompatible drug combinations result in increased side effects, unwanted drug interactions or new side effects.

Moreover, not all combinations of analgesics and glucosamine are suitable, in terms of safety or efficacy for the administration of a single product. Thus, no orally-administrable pharmaceutical combination has been produced until today, which contains a combination of flurbiprofen, glucosamine and capsaicin. Even if some medicaments comprising either of these active agents have been administered concomitantly in practice, this fact requires the patients to carry more than one drug and causes application-related difficulties. Additionally, administering and formulating a combination, in place of the individual use of each active agent, may provide improved treatment features.

Another problem is related to combine these three active ingredients in one dosage form such as tablet or capsule, it would require a dosage form having approximately or more than 1000 mg active ingredients in total without any further tablet or capsule excipients. This is an amount that would create a very large tablet or capsule size that would not be swallowable, or it would require combination that would require ingesting multiple tablets to achieve the desired effect.

Accordingly, based on said drawbacks, a novelty is required in the art of pharmaceutical combinations having therapeutic effects against pain and inflammatory symptoms associated with joint and cartilage disorders, especially with osteoarthritis and rheumatoid arthritis.

Therefore, the object of the present invention is to provide new pharmaceutical combinations comprising flurbiprofen, glucosamin and capsaicin for use in the treatment of pain and inflammatory symptoms associated with joint and cartilage disorders, especially with osteoarthritis and rheumatoid arthritis.

### Detailed description of the invention

The main object of the present invention is to treat, reduce, or prevent the degenerative joint and cartilage disorders by administering to a subject in need thereof a therapeutically effective amount of a combination comprising flurbiprofen, glucosamine and capsaicin , for oral administration, which overcomes the above described problems in prior art and have additional advantages over them.

A further object of the invention is to eliminate the GI adverse effects of flurbiprofen when it is administered orally in high terapeutic effective amounts for a long time. It is known that the treatment of the degenerative joint and cartilage disorders, especially osteoarthritis and rheumatoid arthritis needs a long treatment period. Therefore to use of flurbiprofen for a long time with high terapeutic effective amounts may increase the possibility of GI adverse effects of flurbiprofen. As a rule, after a long-term administration of a drug, drug addiction develops and as a consequence its dosage should be increased. This certainly affects the occurrence of side effects.

The present invention provides the solution to this problem by using not more than 15 % flurbiprofen by combining it with glucosamin not less than 45 % by weight and not more than 15% capsaicin by weight. It has been found surprisingly that this ratios have an increased/synergistic effect over the flurbiprofen's analgesic and antiinflammatory activity even with low doses.

Accordingly, when flurbiprofen is used for a long period of time, it may have a desensitising effect. It has been also found that when flurbiprofen is used in an amount of not more than 15 % by combination with glucosamin not less than 45 % by weight and not more than 15% capsaicin by weight makes it possible to ensure increased analgesic and anti-inflammatory effect of the combination, whilst reducing the pain and inflammation syndrome in degenerative joint and cartilage disorders synernisticly. Thus this also reducer the risk of the GI side effects. In one embodiment flurbiprofen amount is present not more than 10 % by weight, glucosamine sulfate amount is present not less than 50% by weight of the total formulation and capsaicin amount is present not less than 1% by weight of the total formulation.

The role which glucosamine sulfate plays in the treatment or prevention of the degenerative joint and cartilage disorders is most likely associated directly its ability to act as the most important substrate for glycosaminoglycans and a basis of hyaluronic acid. A successful treatment of osteoarthritis and rheumatoid arthritis must control pain effectively as well as slow down or ensure the reverse development of joint degeneration process. It has been found that the introduction of glucosamone sulfate in a quantity of not less than 45 % of the total weight of the combination makes it possible to ensure a chondroprotective and anti-inflammatory effect of the combination and prevents destructive effect of glucocorticoids on chondrocytes and to reduce a need for NSAID (i.e. flurbiprofen) in high dosage for patients suffering from osteoarthritis and rheumatoid arthritis which in turn makes it possible to decrease side effect risks.

These ratios also ensure the required effective doses for the therapy without the need of taking the medicine three times a day. In prior art, the formulations comprising glucosamine are taken three times a day. Due to increased tablet weight when trying to increase the required glucosamine effective doses (i.e 750 mg to 1000 mg/tablet or capsule) which should be the minimum 500 mg, occurs some problems during the manufacturing of the combination itself, and for the patient compliance too. Because it would require a dosage form having approximately or more than 1000 mg active ingredients in total without any further tablet or capsule excipients. This is an amount that would create a very large tablet or capsule size that would not be swallowable, or it would require combination that would require ingesting multiple tablets to achieve the desired effect which can be difficult for the patients.

Therefore it has been found that in certain ratios of flurbiprofen to glucosamine which is in the range of 0.010 to 10.0(w/w), preferably 0.10 to 5.0 (w/w), and more preferably 0.10 to 2.0 (w/w) and flurbiprofen to capsaicin which is in the range of 1.0 to 15.0 (w/w), preferably 1.0 to 10.0 (w/w), and more preferably 2.0 to 5.0 (w/w) helps the combination easily processed into a tablet or capsule dosage form, in desired weight which can easily be swallowed by the patients, whilst maintaining or increasing the therapeutic effective doses for the treatment of joint and cartilage disorders. According to a preferred embodiment of the present invention, said novelty is realized with the combination comprising, flurbiprofen or a pharmaceutically acceptable salt thereof, glucosamine or salts thereof and capsaicin or salts thereof

Flurbiprofen useful in accordance with this invention comprises the pharmaceutically acceptable salts and esters of flurbiprofen, and further includes the conventionally used racemic mixture which comprises the S- and R- enantiomers of flurbiprofen. In a preferred embodiment of the present invention, flurbiprofen is in an amount of 5.0 to 15.0 % by weight of the total tablet, preferably it is 5.0 to 10.0 % by weight of the total tablet.

The preferred salts of glucosamine in accordance with this invention comprise N-acetyl-glucosamine, glucosamine hydrochloride and glucosamine sulfate and mixtures thereof. In a preferred embodiment of the present invention, the salt is sulfate salt. Glucosamine sulfate thereof is in an amount of 45.0 to 70.0 % by weight of the total tablet, preferably it is 50.0 to 70.0 % by weight of the total tablet, more preferably it is 60.0 % to 70.0 % by weight of the total tablet.

Capsaicin has been used in this present invention to increase the analgesic effect of flurbiprofen and glucosamine combination. Capsaicin is a TRPV1 channel agonist indicated for the management of neuropathic pain associated with postherpetic neuralgia. It triggers the release of endorphins from the pituitary gland and hypothalamus which help relieve pain. It has been found that the introduction of capsaicin in an amount of 1.0 - 15.0% and more preferably 1.0-10.0% of the total weight of formulation ensures the improved treatment of joint and cartilage disorders.

In one embodiment, the ratios of glucosamine to capsaicin is in the range of 1.0 to 70.0(w/w), preferably 4.0 to 50.0 (w/w) and more preferably 4.0 to 25.0 (w/w).

However, it also has some side effects including burning sensation in the stomach, lips, tongue and throat. To improve the taste and mask this bitter taste of capsaicin on formulation taste, sweetener and optionally taste masking coating has been used in an effective amount.

In a preferred embodiment of the present invention comprises at least one or more excipient.

According to a preferred embodiment of the present invention, said excipient comprises at least one or more sweeteners, diluents, disintegrants, glidants, lubricants, binders, coloring agents, flavouring agents.

The sweetener is in the range of 0.1 to 8.0% by weight of total formulation; preferably it is 0.1 to 3.0% by weight of total formulation.

In another preferred embodiment, it has been found that in certain ratios of capsaicin to sweetener which is in the range of 0.010 to 50.0 (w/w), preferably 0.10 to 25.0 (w/w) and more preferably 1.0 to 10.0 (w/w) helps to mask the taste of capsaicin in the formulation of this present invention.

In a preferred embodiment of the present invention suitable sweeteners are selected from the group comprising sucralose, sodium cyclamate, thaumatin, mogroside, inuline, erythritol, glycyrrhizin, monosodium glycyrrhizinate, monoamonium glycyrrhizinate, isomalt, glycerine, dextrose or mixtures thereof According to one embodiment of the present invention, the present invention further comprises at least one or more taste masking coating agent.

In further embodiment, taste-masking coating agent has been used during the process, to overcome bitter taste of capsaicin. The taste-masking coating agent comprising aminoalkyl metacrylate, dimethylaminoethyl methacrylate, butyl methacrylate carboxymethylcellulose calcium, copolymer of dimethylaminoethyl methacrylate, butyl methacrylate and methyl methacrylate (Eudragit E 100), mixture of polyethylene glycol and polyvinyl alcohol (Kollicoat IR), carboxymethylcellulose sodium, carnauba wax, cellulose acetate, cellulose acetate phthalate, ceresin, cetyl alcohol, chitosan, ethylcellulose, fructose, gelatin, glycerin, glyceryl behenate, glyceryl palmitostearate, hydroxyethyl cellulose, hydroxyethylmethyl cellulose, hydroxypropyl cellulose, hypromellose, hypromellose phthalate, isomalt, liquid glucose, maltitol, maltodextrin, methylcellulose, microcrystalline wax, paraffin, poloxamer, polydextrose, polyethylene glycol, polyethylene oxide, poly-DL-(lactic acid), polyvinyl acetate phthalate, polyvinyl alcohol, potassium chloride, povidone, shellac, shellac with stearic acid, sucrose, surface color agents, titanium oxide, tributyl citrate, triethyl citrate, vanillin, white wax,sugar, xylitol, yellow wax, zein or their copolymers or their mixtures.

In a preferred embodiment of the present invention, the taste-masking coating agent is in the range of 1.0 to 50.0% by weight of total formulation; preferably it is 2 to 30.0% by weight of total formulation.

In a preferred embodiment of the present invention, suitable diluents is selected from a group comprising lactose monohydrate, microcrystalline cellulose, corn starch, pregelatinized starch, mannitol, calcium phosphate anhydrate, calcium phosphate dihydrate, calcium phosphate trihydrate, dibasic calcium phosphate, calcium carbonate, calcium sulfate, carboxymethyl cellulose calcium, powdered cellulose, cellulose acetate or mixtures thereof.

In a preferred embodiment of the present invention, suitable disintegrant is selected from a group comprising croscarmellose sodium, hydroxypropyl cellulose, xylitol, crospovidone, low-substituted hydroxypropyl cellulose (L-HPC) and sodium starch glycolate, corn starch or mixtures thereof. In one aspect, disintegrant is present in an amount of from 5.0 to 25.0 % by weight of the total tablet formulation.

In a preferred embodiment of the present invention, suitable glidant is colloidal silicon dioxide or talc. In one aspect, glidant is present in an amount of from 0.10 to 5.0 % by weight of the total tablet formulation.

In a preferred embodiment of the present invention, suitable lubricant is selected from the group comprising magnesium stearate, sodium stearyl fumarate, polyethylene glycol, stearic acid, metal stearates, boric acid, sodium chloride benzoate and acetate, sodium or magnesium lauryl sulfate or mixtures thereof. In one aspect, lubricant is present in an amount of from 0.10 to 5.0 % by weight of the total tablet formulation.

In a preferred embodiment of the present invention, suitable binder is selected from a group comprising polymethacrylate, glyceryl behenate, polyvinylpyrrolidone (povidone), hydroxypropyl methyl cellulose (HPMC), hydroxypropyl cellulose (HPC), carboxymethyl cellulose (CMC), methyl cellulose (MC), hydroxyethyl cellulose, sodium carboxymethyl cellulose (Na CMC), carboxymethyl cellulose calcium, ethyl cellulose and other cellulose derivatives, polyethylene oxide, gelatin, starch, xanthan gum, guar gum, alginate, carrageen, pectin, carbomer, cellulose acetate phthalate, hydroxypropyl starch, hydroxyethyl methyl cellulose, polaxomer, polyethylene glycol (PEG) or mixtures thereof. In one aspect, binder is used optionally and may present in an amount of from 0.10 to 10.0 % by weight of the total tablet formulation.

In a preferred embodiment of the present invention, suitable coloring agent is selected from a group comprising iron oxides (such as; iron oxide yellow, red or black), Food, Drug & Cosmetic (FD&C) dyes, poncau, indigo blue, indigotine blue, carmoisine indigotine, quinoline yellow, flaming red, carmine, carmoisine, sunset yellow or mixtures thereof. In one aspect, coloring agent is used optionally and may present in an amount of from 0.01 to 1.00 % by weight of the total tablet formulation.

In a preferred embodiment of the present invention, suitable flavouring agent is selected from a group comprising fruit flavours such as orange, banana, strawberry, cherry, wild cherry, lemon; and other flavours such as cardamom, anise, peppermint, menthol, vanillin and ethyl vanillin or mixtures thereof. In one aspect, flavouring agent is used optionally and may present in an amount of from 0.1 to 2.0 by weight of total formulation.

In a preferred embodiment according to the present invention, said pharmaceutical combination comprises,
a. flurbiprofen at 5.0 - 15.0 % by weight,
b. glucosmain sulfate at 45.0 - 70.0 % by weight,
c. capsaicin at 1.0 - 15 % by weight,
d. lactose monohydrate at 10.0 - 20 % by weight,
e. microcrystalline cellulose at 5.0 - 10 % by weight,
f. croscarmellose sodium at 1.0 - 5.0 % by weight,
g. hydroxypropyl cellulose at 1.0 - 5.0 % by weight,
h. colloidal silicon dioxide at 0.10 - 2.0 % by weight,
i. magnesium stearate at 0.10 - 2.0 % by weight,
j. sweetener 0.1 - 3.0 % by weight,
k. optionally, taste-masking coating agent.

In another preferred embodiment according to the present invention, said pharmaceutical combination comprises,
a. flurbiprofen at 5.0 - 10.0 % by weight,
b. glucosmain sulfate at 50.0 - 70.0 % by weight,
c. capsaicin at 1.0 - 10 % by weight,
d. lactose monohydrate at 10.0 - 20 % by weight,
e. microcrystalline cellulose at 5.0 - 10 % by weight,
f. croscarmellose sodium at 1.0 - 3.0 % by weight,
g. hydroxypropyl cellulose at 1.0 - 3.0 % by weight,
h. colloidal silicon dioxide at 0.10 - 1.0 % by weight,
i. magnesium stearate at 0.10 - 1.0 % by weight,
l. sweetener 0.1 - 3.0 % by weight,
m. optionally, taste-masking coating agent.

According to another preferred embodiment of the present invention, the flurbiprofen or a pharmaceutically acceptable salts thereof combinations comprising glucosamine is used in the treatment of pain and inflammatory symptoms associated with joint and cartilage disorders, especially with osteoarthritis and rheumatoid arthritis.

According to another embodiment of the invention, the pharmaceutical combination is in the form of a tablet or capsule, it may optionally in the form of a bilayer or multilayer tablet.

As a further embodiment of the invention, it is possible to prepare tablets or granules by direct compression. Likewise the dry and wet granulation processes are possible as well.

The preferred direct compression process of the present invention for preparing the pharmaceutical combination comprises the following steps;
a. blending flurbiprofen, glucosamine sulphate, capsaicin, microcrystalline cellulose, lactose monohydrate, crosscarmellose sodium and hydroxypropylcellulose progressively, wherein the blending time is preferably 20 min.,
b. adding magnesium stearate, colloidal silicon dioxide and sweetener to the powder mixture above and blending progressively for about 5 min.
c. compressing the final powder mixture to form tablets, or filling into capsules,
d. optionally, taste-masking coating agent said tablets.

The preferred dry granulation process of the present invention for preparing the pharmaceutical combination comprises the following steps;
a. mixing flurbiprofen, glucosamine sulfate and capsaicin with lactose monohydrtate, ½ of microcrystalline cellulose, crosscarmellose sodium and hydroxypropylcellulose and granulating the mixture progressively,
b. compacting the blended mixture,
c. adding rest of the microcrystalline cellulose, colloidal silicon dioxide, magnesium stearate and sweetener to this mixture of step b., and further progressive blending until obtaining a homogenous powder mixture,
d. compressing the blended mixture to form tablets or filling into capsules,
e. optionally, taste-masking coating agent said tablets.

The preferred wet granulation process for preparing the pharmaceutical combination comprising the following steps;
a. mixing flurbiprofen, glucosamine sulfate and capsaicin with lactose monohydrtate, microcrystalline cellulose, ½ of crosscarmellose sodium and hydroxypropylcellulose and blending,
b. adding water or water + alcohol mixture to this mixture and blending to form granules,
c. sieving and drying the wet granules in oven or fluid bed dryer and sieving the dry granules,
d. rest of the crosscarmellose sodium, colloidal silicon dioxide, magnesium stearate and sweetener are mixed with the dry granule mixture,
e. compressing the blended mixture to form tablets, or filling into capsules,
f. optionally, taste-masking coating agent said tablets.
This invention is further defined by reference to the following examples. Although the example is not intended to limit the scope of the present invention, it should be considered in the light of the description detailed above.

**Example 1: capsul or tablet**

| **Ingredients** | **% amount** |
|---|---|
| flurbiprofen | 8.70 |
| glucosamin sulfate | 65.20 |
| capsaicin | 3.00 |
| lactose monohydrate | 15.30 |
| microcrystalline cellulose | 4.00 |
| croscarmellose sodium | 1.00 |
| hydroxypropyl cellulose | 1.50 |
| colloidal silicon dioxide | 0.30 |
| magnesium stearate | 0.30 |
| **sweetener** | 0.7 |

The process of the combination is carried out with one of the processes as given above in detail with the optional taste masking coating agent.

## Claims

1. A pharmaceutical combination for oral administration, comprising flurbiprofen or a pharmaceutically acceptable salt thereof, glucosamine sulfate and capsaicin or a pharmaceutically acceptable salt thereof; wherein the amount of glucosamine sulfate is not less than 45 % and preferably between 45 % and 70 % by weight of the total formulation.

2. The pharmaceutical combination according to claim 1, wherein the amount of flurbiprofen or pharmaceutically acceptable salts thereof is not more than 15 % by weight of the total formulation.

3. The pharmaceutical combination according to 1, wherein the amount of capsaicin is not more than 15%, preferably in the range of 1.0 - 15.0% and more preferably 1.0-10.0% of the total weight of formulation.

4. The pharmaceutical combination according to claims 1 to 3, wherein the ratio of flurbiprofen to glucosamine which is in the range of 0.010 to 10.0(w/w), preferably 0.10 to 5.0 (w/w), and more preferably 0.10 to 2.0 (w/w)

5. The pharmaceutical combination according to claims 1 to 4, wherein the ratio of flurbiprofen to capsaicin is in the range of 1.0 to 15.0 (w/w), preferably 1.0 to 10.0 (w/w), and more preferably 2.0 to 5.0 (w/w).

6. The pharmaceutical combination according to claims 1 to 5, wherein the ratio of glucosamine to capsaicin is in the range of 1.0 to 70.0(w/w), preferably 4.0 to 50.0 (w/w) and more preferably 4.0 to 25.0 (w/w).

7. The pharmaceutical combination according to claims 1 to 6, comprising at least one or more pharmaceutically acceptable excipient.

8. The pharmaceutical combination according to claim 7 wherein said excipient comprises at least one or more sweeteners, diluents, disintegrants, glidants, lubricants, binders, coloring agents, flavouring agents.

9. The pharmaceutical combination according to claims 8, wherein the amount of sweetener is in the range of 0.1 to 8.0% by weight of total formulation; preferably it is 0.1 to 3.0% by weight of total formulation.

10. The pharmaceutical combination according to any preceding claim, wherein the ratio of capsaicin to sweetener is in the range of 0.010 to 50.0 (w/w), preferably 0.10 to 25.0 (w/w) and more preferably 1.0 to 10.0 (w/w).

11. The pharmaceutical combination according to any preceding claim, further comprising at least one or more taste masking coating agent.

12. The pharmaceutical combination according to any preceding claim, wherein the amount of taste-masking coating agent is in the range of 1.0 to 50.0% by weight of total formulation; preferably it is 2 to 30.0% by weight of total formulation.

13. The pharmaceutical combination according to any preceding claim, comprising,
a. flurbiprofen at 5.0 - 15.0 % by weight,
b. glucosamine sulfate at 45.0 - 70.0 % by weight,
c. capsaicin at 1.0 - 15 % by weight,
d. lactose monohydrate at 10.0 - 20 % by weight,
e. microcrystalline cellulose at 5.0 - 10 % by weight,
f. croscarmellose sodium at 1.0 - 5.0 % by weight,
g. hydroxypropyl cellulose at 1.0 - 5.0 % by weight,
h. colloidal silicon dioxide at 0.10 - 2.0 % by weight,
i. magnesium stearate at 0.10 - 2.0 % by weight,
j. sweetener 0.1 - 3.0 % by weight,
k. optionally, taste-masking coating agent,

14. The pharmaceutical combination according to any preceding claim, comprising,
a. flurbiprofen at 5.0 - 10.0 % by weight,
b. glucosamine sulfate at 50.0 - 70.0 % by weight,
c. capsaicin at 1.0 - 10 % by weight,
d. lactose monohydrate at 10.0 - 20 % by weight,
e. microcrystalline cellulose at 5.0 - 10 % by weight,
f. croscarmellose sodium at 1.0 - 3.0 % by weight,
g. hydroxypropyl cellulose at 1.0 - 3.0 % by weight,
h. colloidal silicon dioxide at 0.10 - 1.0 % by weight,
i. magnesium stearate at 0.10 - 1.0 % by weight,
a. sweetener 0.1 - 3.0 % by weight,
b. optionally, taste-masking coating agent.

15. The pharmaceutical combination according to any preceding claim, wherein the combination is in the form of a capsule, tablet bilayer tablet or multilayer tablet.

## Patentansprüche

1. Pharmazeutische Kombination zur oralen Verabreichung, umfassend Flurbiprofen oder ein pharmazeutisch unbedenkliches Salz davon, Glucosaminsulfat und Capsaicin oder ein pharmazeutisch unbedenkliches Salz davon; wobei die Menge an Glucosaminsulfat - auf das Gewicht der Gesamtrezeptur bezogen - nicht unter 45 %, und vorzugsweise zwischen 45 % und 70%, liegt.

2. Pharmazeutische Kombination nach Anspruch 1, bei der die Menge an Flurbiprofen oder dessen pharmazeutisch unbedenklicher Salze - auf das Gewicht der Gesamtrezeptur bezogen - nicht mehr als 15 % beträgt.

3. Pharmazeutische Kombination nach Anspruch 1, bei der die Menge an Capsaicin - auf das Gewicht der Gesamtrezeptur bezogen - nicht mehr als 15 % beträgt, wobei es vorzugsweise im Bereich von 1,0 % bis 15,0 %, und noch bevorzugter von 1,0 % bis 10,0 %, liegt.

4. Pharmazeutische Kombination nach den Ansprüchen 1 bis 3, bei der das Verhältnis von Flurbiprofen zu Glucosamin im Bereich von 0,010 bis 10,0 (auf das Gewicht bezogen), vorzugsweise von 0,10 bis 5,0 (auf das Gewicht bezogen), und noch bevorzugter von 0,10 bis 2,0 (auf das Gewicht bezogen) liegt.

5. Pharmazeutische Kombination nach den Ansprüchen 1 bis 4, bei der das Verhältnis von Flurbiprofen zu Capsaicin im Bereich von 1,0 bis 15,0 (auf das Gewicht bezogen), vorzugsweise von 1,0 bis 10,0 (auf das Gewicht bezogen), und noch bevorzugter von 2,0 bis 5,0 (auf das Gewicht bezogen) liegt.

6. Pharmazeutische Kombination nach den Ansprüchen 1 bis 5, bei der das Verhältnis von Glucosamin zu Capsaicin im Bereich von 1,0 bis 70,0 (auf das Gewicht bezogen), vorzugsweise von 4,0 bis 50,0 (auf das Gewicht bezogen), und noch bevorzugter von 4,0 bis 25,0 (auf das Gewicht bezogen) liegt.

7. Pharmazeutische Kombination nach den Ansprüchen 1 bis 6, umfassend mindestens einen oder mehrere pharmazeutisch unbedenkliche(n) Hilfsstoff(e).

8. Pharmazeutische Kombination nach Anspruch 7, bei der der Hilfsstoff mindestens ein(en) oder mehrere Süßstoff(e), Verdünnungsmittel, Sprengmittel, Fließregulierungsmittel, Gleitmittel, Bindemittel, Farbstoff(e), Aromastoff(e) umfasst.

9. Pharmazeutische Kombination nach Anspruch 8, bei der die Menge an Süßstoff im Bereich von 0,1 % bis 8,0 % des Gewichts der Gesamtrezeptur liegt, vorzugsweise im Bereich von 0,1 % bis 3,0 % des Gewichts der Gesamtrezeptur beträgt.

10. Pharmazeutische Kombination nach einem der voranstehenden Ansprüche, bei der das Verhältnis von Capsaicin zu Süßstoff im Bereich von 0,010 bis 50,0 (auf das Gewicht bezogen), vorzugsweise von 0,10 bis 25,0 (auf das Gewicht bezogen), und noch bevorzugter von 1,0 bis 10,0 (auf das Gewicht bezogen) liegt.

11. Pharmazeutische Kombination nach einem der voranstehenden Ansprüche, des Weiteren umfassend mindestens ein oder mehrere Überzugsmittel zur Geschmacksmaskierung.

12. Pharmazeutische Kombination nach einem der voranstehenden Ansprüche, bei der die Menge an Überzugsmittel zur Geschmacksmaskierung im Bereich von 1,0 % bis 50,0 % des Gewichts der Gesamtrezeptur liegt; vorzugsweise von 2,0 % bis 30,0 % des Gewichts der Gesamtrezeptur beträgt.

13. Pharmazeutische Kombination nach einem der voranstehenden Ansprüche, umfassend:
a. Flurbiprofen zu 5,0 bis 15,0 Gew.-%,
b. Glucosaminsulfat zu 45,0 bis 70,0 Gew.-%,
c. Capsaicin zu 1,0 bis 15,0 Gew.-%,
d. Laktosemonohydrat zu 10,0 bis 20,0 Gew.-%,
e. mikrokristalline Cellulose zu 5,0 bis 10,0 Gew.-%,
f. Croscarmellose-Natrium zu 1,0 bis 5,0 Gew.-%,
g. Hydroxypropylcellulose zu 1,0 bis 5,0 Gew.-%,
h. kolloidales Siliziumdioxid zu 0,10 bis 2,0 Gew.-%,
i. Magnesiumstearat zu 0,10 bis 2,0 Gew.-%,
j. Süßstoff zu 0,1 bis 3,0 Gew.-%,
k. fakultativ, Überzugsmittel zur Geschmacksmaskierung.

14. Pharmazeutische Kombination nach einem der voranstehenden Ansprüche, umfassend:
a. Flurbiprofen zu 5,0 bis 10,0 Gew.-%,
b. Glucosaminsulfat zu 50,0 bis 70,0 Gew.-%,
c. Capsaicin zu 1,0 bis 10,0 Gew.-%,
d. Laktosemonohydrat zu 10,0 bis 20,0 Gew.-%,
e. mikrokristalline Cellulose zu 5,0 bis 10,0 Gew.-%,
f. Croscarmellose-Natrium zu 1,0 bis 3,0 Gew.-%,
g. Hydroxypropylcellulose zu 1,0 bis 3,0 Gew.-%,
h. kolloidales Siliziumdioxid zu 0,10 bis 1,0 Gew.-%,
i. Magnesiumstearat zu 0,10 bis 1,0 Gew.-%,
j. Süßstoff zu 0,1 bis 3,0 Gew.-%,
k. fakultativ, Überzugsmittel zur Geschmacksmaskierung.

15. Pharmazeutische Kombination nach einem der voranstehenden Ansprüche, bei der die Kombination in Form einer Kapsel, einer Tablette, einer Zweischichttablette oder einer Mehrschichttablette vorliegt.

## Revendications

1. Combinaison pharmaceutique pour administration orale, comprenant du flurbiprofène ou un sel de celui-ci, de qualité pharmaceutique, du sulfate de glucosamine et de la capsaïcine, ou un sel de ceux-ci de qualité pharmaceutique ; dans laquelle la quantité de sulfate de glucosamine ne doit pas être inférieure à 45 % et doit se situer de préférence entre 45 % et 70 % en poids de la formulation totale.

2. Combinaison pharmaceutique selon la revendication 1, dans laquelle la quantité de flurbiprofène, ou de sels de celui-ci de qualité pharmaceutique, n'est pas supérieure à 15 % en poids de la formulation totale.

3. Combinaison pharmaceutique selon la revendication 1, dans laquelle la quantité de capsaïcine n'est pas supérieure à 15 %, se situant de préférence dans une fourchette allant de 1.0 à 15.0 % et encore mieux, dans une fourchette allant de 1.0 à 10.0 % du poids total de la formulation.

4. Combinaison pharmaceutique selon les revendications 1 à 3, dans laquelle le taux de flurbiprofène par rapport à la glucosamine se trouve dans une fourchette allant de 0.010 à 10.0 (P/P), de préférence de 0.10 à 5.0 (P/P) et encore mieux, de 0.10 à 2.0 (P/P).

5. Combinaison pharmaceutique selon les revendications 1 à 4, dans laquelle le rapport de flurbiprofène par rapport à la capsaïcine se trouve dans une fourchette allant de 1.0 à 15.0 (P/P), de préférence de 1.0 à 10.0 (P/P) et encore mieux, de 2.0 à 5.0 (P/P).

6. Combinaison pharmaceutique selon les revendications 1 à 5, dans laquelle le rapport de la glucosamine par rapport à la capsaïcine se trouve dans une fourchette allant de 1.0 à 70.0 (P/P), de préférence de 4.0 à 50.0 (P/P) et encore mieux, de 4.0 à 25.0 (P/P).

7. Combinaison pharmaceutique selon les revendications 1 à 6, comprenant au moins un ou plusieurs excipients de qualité pharmaceutique.

8. Combinaison pharmaceutique selon la revendication 7, dans laquelle ledit excipient comprend au moins un ou plusieurs édulcorants, diluants, agents délitant, agents de glissement, lubrifiants, liants, colorants, agents aromatisant.

9. Combinaison pharmaceutique selon la revendication 8, dans laquelle la quantité d'édulcorant se situe dans une fourchette allant de 0.1 à 8.0 % en poids de la formulation totale ; de préférence de 0.1 à 3.0 % en poids de la formulation totale.

10. Combinaison pharmaceutique selon l'une quelconque des revendications précédentes, dans laquelle le rapport entre la capsaïcine et l'édulcorant se situe dans une fourchette allant de 0.010 à 50.0 % (P/P), de préférence de 0.10 à 25.0 (P/P) et encore mieux, de 1.0 à 10.0 (P/P).

11. Combinaison pharmaceutique selon l'une quelconque des revendications précédentes comprenant, en outre, au moins un ou plusieurs agents d'enrobage masquant le goût.

12. Combinaison pharmaceutique selon l'une quelconque des revendications précédentes, dans laquelle la quantité d'agent d'enrobage masquant le goût se trouve dans une fourchette comprise entre 1.0 et 50.0 % en poids de la formulation totale ; de préférence entre 2 et 30.0 % en poids de la formulation totale.

13. Combinaison pharmaceutique selon l'une quelconque des revendications précédentes, comprenant,
a. du flurbiprofène à 5.0 à 15.0 % en poids,
b. du sulfate de glucosamine à 45.0 - 70.0 % en poids,
c. de la capsaïcine à 1.0 - 15 % en poids
d. du monohydrate de lactose à 10.0 - 20 % en poids
e. de la cellulose microcristalline à 5.0 - 10 % en poids,
f. de la croscarmellose sodique à 1.0 - 5.0 % en poids
g. de l'hydroxyprolyl cellulose à 1.0 - 5.0 % en poids
h. de la silice sublimée à 0.10 - 20 % en poids,
i. du stéarate de magnésium à 0.10 - 25.0 % en poids,
j. de l'édulcorant à 0.1 - 3.0 % en poids,
k. en option, un agent d'enrobage masquant le goût,

14. Combinaison pharmaceutique selon l'une quelconque des revendications précédentes, comprenant,
a. du flurbiprofène à 5.0 - 10 % en poids
b. du sulfate de glucosamine à 50.0 - 70.0 % en poids,
c. de la capsaïcine à 1.0 - 10 % en poids,
d. du monohydrate de lactose à 10.0 - 20 % en poids,
e. de la cellulose microcristalline à 5.0 - 10 % en poids,
f. de la croscarmellose sodique à 1.0 - 3.0 % en poids
g. de l'hydroxyprolyl cellulose à 1.0 - 3.0 % en poids
h. de la silice sublimée à 0.10 - 1.0 % en poids,
i. du stéarate de magnésium à 0.10 - 1.0 % en poids,
a. de l'édulcorant à 0.1 - 3.0 % en poids,
b. en option, un agent d'enrobage masquant le goût.

15. Combinaison pharmaceutique selon l'une quelconque des revendications précédentes, dans laquelle la combinaison se trouve sous la forme d'une capsule, d'un comprimé, d'un comprimé bicouches ou d'un comprimé multicouches.
